# EUROPEAN PATENT SPECIFICATION

(11) **EP 1 315 526 B1**
(45) Date of publication and mention of the grant of the patent: **24.11.2004**
(21) Application number: 01993154.2
(22) Date of filing: 06.09.2001
(51) Int. Cl.: A61L 9/01, A61L 9/014, A61L 9/16, A61K 7/32, A01K 1/00

(54) **COMPOSITIONS AND METHODS FOR REDUCING ODOR**
ZUSAMMENSETZUNG UND METHODE ZUR GERUCHSVERRINGERUNG
COMPOSITIONS ET PROCEDES PERMETTANT D'ATTENUER LES ODEURS

(30) Priority: 07.09.2000 US 232151 P
(43) Date of publication of application: 04.06.2003
(73) Proprietor: Solutia Inc., St. Louis, Missouri 63141 (US)
(72) Inventor: HOCHWALT, Mark, A., St. Louis, MO 63107 (US); TREHY, Michael, L., St. Louis, MO 63146 (US)
(74) Representative: Albrecht, Thomas, Dr.
(86) International application number: PCT/US2001/042060
(87) International publication number: WO 2002/055115

(56) References cited:
- WO-A-00/23120
- WO-A-91/12029
- WO-A-98/27261
- US-A- 5 911 976

## Description

### FIELD OF THE INVENTION

This invention relates to compositions and methods effective for reducing odor. In one embodiment, this invention relates to compositions and methods effective for reducing a wide range of odors using an effective amount of a composition(s) comprising effective amounts of at least one acid, at least one synthetic zeolite and at least one metal, metal oxide, or salt of a metal or metal oxide.

### BACKGROUND OF THE INVENTION

Odors offensive to the human olfactory system originate from a variety of sources including humans and animals, such as pet and animal wastes, and from activities, such as from cooking and from tobacco smoke. Some of these offensive odors can be attributed to odors from specific chemicals like methyl mercaptan, hydrogen sulfide, methyl sulfide, methyl disulfide, trimethylamine, acetaldehyde, n-butyric acid, n-valeric acid, iso-valeric acid and propionic acid and the like. Many of these aforementioned chemicals can arise from partial decomposition of animal and plant wastes. Other odors that are sometimes found to be offensive to the human olfactory system can originate from foods such as garlic and onions either when such foods are fresh or stale due to spoilage. The odors associated with this wide range of sources are complex and can consist of a mixture of different substances including, but not limited to, inorganic bases such as ammonia, organic acids such as butyric acid and neutral organic molecules such as allicin (odor of garlic).

Many compositions and methods have been developed for the purpose of eliminating or controlling odor. However, these methods and compositions have had only limited success because the agents typically used for such purposes either are effective at controlling only a limited range of odors, or control odors by masking them with other odors (fragrances) or have other limitations.

For example, one conventional method of masking such odors often involves the use of fragrances or perfumes. However, masking has disadvantages. Masking does not remove the components of the odors itself, and for many, the masking odor itself is unpleasant. In addition, many odor reducing or masking agents are specific and not effective for controlling all odors. Specifically, amine and ammonia odors are difficult to mask with fragrances.

Sorbants like activated carbon and natural and synthetic zeolites are effective for trapping many odors, but the use of both have disadvantages. For example, activated carbon is unsuitable for use where the sorbant(s) may come in contact with animals, furniture, or carpets, while natural and most synthetic zeolites have low capacities for odors in the presence of moisture. Natural zeolites typically trap odors. However, the capacity of natural zeolites is very limited due to natural zeolites stronger affinity for water than for the odor molecules. Natural zeolites are often used to release fragrances on exposure to moisture rather than to trap odors.

Another method of odor removal is by chemical reaction(s). Chemicals like acids and bases can be used to trap the base and acid components present that cause odors. Acids like butyric acid and hydrogen sulfide can be trapped by bases like sodium bicarbonate (baking soda) while basic compounds like ammonia can be trapped by acids like phosphoric acid. In order to maximize their performance, generally the acid and base components are separated into different traps and the odors are treated sequentially. The use of acids and bases to trap odors is incomplete because these sorbants do not trap neutral odors such as allicin. Consequently, the use of acids and bases alone is insufficient.

In addition to the use of acids and bases for odor removal, metals, metal oxides and metal salts have been used for control of odors by chemical interactions. In this case, transition metals like copper are particularly effective in trapping odors due to sulfides by forming non-odorous metal complexes with the sulfides. Again, the metals have limited coverage and are ineffective against neutral molecules and ammonia odors.

Despite the foregoing, need continues to exist in the art to provide a composition and method for effectively reducing odor that is odorless and effectively controls a wide range of odors, including ammonia, amines, sulfur bearing compounds and other malodorous materials, e.g. fatty acids, which are generated from pet litter, household activities and wastes and the like.

### OBJECTS OF THE INVENTION

It is an object of the present invention to provide safe and effective novel compositions for reducing a wide range of odors, including ammonia, sulfurous odors and neutral organics, which are non-toxic and safe for direct or indirect human or animal contact.

It is a further object of the invention to provide a method for reducing a wide range of odors using the odor reducing composition(s) of this invention.

It is a still further object of the invention to provide odor controlling articles containing the odor reducing compositions of the invention that are effective at controlling odors emitted from the articles during use.

The above and other objects are met in the invention which is described in more non-limiting detail hereinafter.

### SUMMARY OF THE INVENTION

According to the invention, and in one embodiment, an odor reducing composition is provided which is either a composition comprising:
(a) 0.1 wt. % to 99.7 wt. % of at least one acid having a pKₐ greater than 2.9 and less than 6, water solubility less than 45 g/100g H₂O, and an oral rat LD₅₀ greater than 2200 mg/kg;
(b) 0.1 wt. % to 99.7 wt. % of at least one synthetic zeolite having at least 90 percent of its tetrahedral oxide units as SiO₂ tetrahedra, a capacity for adsorbed water of not greater than 10 weight percent when measured at 25 °C and water vapor pressure at 4.6 torr, and pore apertures at least 5.5 Å in diameter, wherein the original water of hydration has been substantially removed; and
(c) 0.1 wt. % to 99.7 wt. % of a substance selected from the group consisting of a metal, metal oxide, and any combination thereof;
wherein the sum of (a), (b), and (c) is 100 wt. %;
or a composition comprising:
(a) 0.1 wt. % to 99.7 wt % of at least one acid selected from adipic, aspartic, cyclohexane-1:1-dicarboxylic, cystine, dimethylmalonic, fumaric, sorbic, glutaric, methylsuccinic, itaconic, or tartaric acid;
(b) 0.1 wt. % to 99.7 wt. % of at least one synthetic zeolite having at least 90 percent of its tetrahedral oxide units as SiO₂ tetrahedra, a capacity for adsorbed water of not greater than 10 weight percent when measured at 25 °C and water vapor pressure at 4.6 torr, and pore apertures at least 5.5 Å in diameter, wherein the original water of hydration has been substantially removed; and
(c) 0.1 wt. % to 99.7 wt. % of a substance selected from the group consisting of a metal, metal oxide, a salt of a metal or metal oxide, and any combination thereof;
wherein the sum of (a), (b), and (c) is 100 wt. %.

In another embodiment of this invention, a method for removing odors from an odor emitting environment is provided comprising contacting an effective amount of the odor reducing composition of the invention with the odor emitting environment
and allowing for a sufficient time to pass for the odor reducing composition to essentially remove the odor.

In yet another embodiment of this invention, a method for removing odor is provided comprising adding an effective amount of the odor reducing composition of the invention to an article that, in the absence of the odor reducing composition, emits an odor during use, and essentially removing the emitted odor from the article.

In a still further embodiment of this invention, an article is provided comprising an article that, in the absence of the odor reducing composition of the invention, emits an odor during use, and the odor reducing composition of the invention in an amount effective at controlling odors emitted from the article during use.

### DETAILED DESCRIPTION OF THE INVENTION

This invention comprises a novel composition for reducing odor. The composition is safe and effective at reducing a broad array of odors and is practically non-toxic. Further, unlike prior odor reducing compositions, the present composition is safe for direct or indirect animal contact. As used herein the term "animal" includes human beings.

The present inventive composition for reducing odor is either a composition comprising:
(a) 0.1 wt. % to 99.7 wt. % of at least one acid having a pKₐ greater than 2.9 and less than 6, water solubility less than 45 g/100g H₂O, and an oral rat LD₅₀ greater than 2200 mg/kg;
(b) 0.1 wt. % to 99.7 wt. % of at least one synthetic zeolite having at least 90 percent of its tetrahedral oxide units as SiO₂ tetrahedra, a capacity for adsorbed water of not greater than 10 weight percent when measured at 25 °C and water vapor pressure at 4.6 torr, and pore apertures at least 5.5 Å in diameter, wherein the original water of hydration has been substantially removed; and
(c) 0.1 wt. % to 99.7 wt. % of a substance selected from the group consisting of a metal, metal oxide, and any combination thereof;
wherein the sum of (a), (b), and (c) is 100 wt. %;
or a composition comprising:
(a) 0.1 wt. % to 99.7 wt % of at least one acid selected from adipic, aspartic, cyclohexane-1:1-dicarboxylic, cystine, dimethylmalonic, fumaric, sorbic, glutaric, methylsuccinic, itaconic, or tartaric acid;
(b) 0.1 wt. % to 99.7 wt. % of at least one synthetic zeolite having at least 90 percent of its tetrahedral oxide units as SiO₂ tetrahedra, a capacity for adsorbed water of not greater than 10 weight percent when measured at 25 °C and water vapor pressure at 4.6 torr, and pore apertures at least 5.5 Å in diameter, wherein the original water of hydration has been substantially removed; and
(c) 0.1 wt. % to 99.7 wt. % of a substance selected from the group consisting of a metal, metal oxide, a salt of a metal or metal oxide, and any combination thereof;
wherein the sum of (a), (b), and (c) is 100 wt. %. In the practice of this invention, the composition of this invention is produced by admixing the readily available components together. This is contrary to prior art odor reducing compositions that describe zeolites coated with metal compounds or serve as a support for metal compounds. Zeolites that are coated with metal compounds, or serve as an inert support for metal compounds, are not suitable for use in the present invention because the metallic coating of zeolites result in at least partial inactivation of the adsorption properties of the zeolite. Accordingly, the zeolite becomes inert, and does not serve an active role in the adsorption of odors as in the present invention.

The invention comprises at least one acid having a pKₐ greater than 2.9 and less than 6, water solubility less than 45 g/100g H₂O, and an oral rat LD₅₀ greater than 2200 mg/kg. Preferably, the acid is essentially odorless. In addition, it is currently preferred that the first pKₐ (pKₐ₁) of the acid is greater than 2.9 and less than 6. In one embodiment, at least one acid is selected from the group consisting of an organic acid, amino acid, and mixtures thereof. Examples of suitable acids useful herein include but are not limited to carboxylic acids, polycarboxylic acids having two or more -COOH groups, amino acids, polyamino acids having two or more -COOH groups, and any suitable combination thereof. Accordingly, the acid may be adipic, polyacryclic, polyaspartic, aspartic, glutamic, mixtures thereof and the like.

Illustrative dicarboxylic acids that may be used in the present invention include, but are not limited to, adipic, aspartic, cyclohexane-1:1-dicarboxylic, cystine, dimethylmalonic, fumaric, sorbic, glutaric, methylsuccinic, itaconic, succinic and tartaric acid, mixtures thereof and the like. Preferred acids useful in this invention include adipic, fumaric, sorbic, aspartic, mixtures of, and the like.

The odor reducing composition(s) of this invention also includes at least one of a metal, metal oxide, a salt of a metal or metal oxide, or any combination thereof. Metal oxides that may be used herein, include but are not limited to, zinc oxide, copper oxide, iron oxide, manganese oxide, tin oxide, silver oxide, mixtures thereof and the like. Preferred metal oxides include zinc oxide, copper oxide and iron oxide, with zinc oxide and copper oxide being most preferred.

In another embodiment, a metal may also be used. Suitable metals according to the invention include, but are not limited to, zinc, copper, iron, manganese, tin and silver. Preferred metals are zinc, iron and copper, with zinc and copper being most preferred.

If desired, a salt of a metal or metal oxide may also be employed in the odor reducing composition(s) of this invention. Any salt of a suitable metal or metal oxide may be used including, but not limited to, salts of zinc, copper, iron, manganese, tin and silver. Preferred salts are salts of zinc or zinc oxide, iron or iron oxide and copper or copper oxide, with salts of zinc, copper, zinc oxide and copper oxide being more preferred. Salts of copper that may by used include, but are not limited to CuSO₄. It is generally desired to use environmentally friendly salts including but not limited to metal sulfates, halides, carbonates, bicarbonates, organic acid salts, mixtures thereof and the like. Examples of salts which are effective in the practice of this invention include zinc sulfate, copper sulfate, zinc chloride, copper chloride, zinc aspartate, mixtures thereof and the like. Zinc salts are most preferred.

At least one zeolite/molecular sieve, is used as an active agent in the present invention. Zeolites useful herein include those zeolites and molecular sieves where sufficient sites and/or pores are available for carrying out this invention so as to provide ample means for adsorption, desorption, diffusion and the like. The zeolite of the odor reducing composition of the invention has an effective number of pores or sites available for the practice of this invention. Zeolites, generally, are three-dimensional, microporous, crystalline solids with well defined structures that contain aluminum, silicon and oxygen in their regular framework. Zeolites are minerals that are mined in many parts of the world and also produced synthetically. Prior art odor reducing compositions have used natural zeolites for odor adsorption. However, natural zeolites have a high affinity for water. Therefore, they are unsuitable in the present invention because the odorous compounds are displaced during use. Natural zeolites are essentially inert when used in the present composition, particularly when such compositions are contacted with water during use. The effective zeolites are those zeolites that have an affinity for organic compounds, but a sufficiently low affinity for water such that there is essentially no displacement of organic compounds. Accordingly, only synthetic zeolites are suitable for the present invention. Therefore, as used herein, the term "zeolite" means synthetic zeolite.

Suitable zeolites useful herein include high-silica zeolites. Examples of high-silica zeolites suitable for the present invention include, but are not limited, to the synthetic high-silica zeolites described in U.S. Patent Nos. 5,013,335 and 4,795,482 to Marcus and Gioffre, et al., which issued on May 7,1991 and January 3,1989, respectively, and which are herein incorporated by reference in their entirety.

In a specific embodiment, the zeolite of the present invention is at least partially activated, at least 90 percent of the framework tetrahedral oxide units are SiO₂ tetrahedra, have pore diameters of at least 5.5 Å and have a capacity for adsorbed water not greater than 10 weight percent when measured at 25° C and at a water vapor pressure of 4.6 torr. In one embodiment, the zeolite is an aluminosilicate having a framework SiO₂/Al₂O₃ molar ratio greater than 35. In an alternate embodiment, the activated zeolite is comprised of a mixture of a silica polymorph and an aluminosilicate having a framework SiO₂/Al₂O₃ molar ratio in the range of about 200 to about 500. Illustratively, commercially available zeolites suitable for use in the present invention include, Zeolyst™ CBV 901, available from Zeolyst International, Valley Forge, PA, USA; and Smellrite®, available from Universal Oil Products, Des Plaines, IL, USA.

The odor reducing composition of the invention comprises an acid in the range of 0.1% to 99.7%, metal, metal oxide or a salt of a metal or metal oxide and any combination thereof, in the range of 0.1% to 99.7%, and synthetic zeolite in the range of 0.1% to 99.7% of the total composition based on the sum of these three components being 100%. In use, the relative ratios of the active components may be tailored as desired for specific odor reduction applications.

In another embodiment the composition of this invention comprises an acid in the range from about 33 to about 99%; metal, oxide or a salt of a metal oxide and any combination thereof in the range from about 0.5 to about 40% and zeolite from about 5% to about 50% based on the sum of three components being 100%.

Preferably, in one other embodiment, the metal, metal oxide, or salt and acid combined are present in the range of about 24% to about 99.7% of the total composition and zeolite comprises in the range of about 0.3% to about 76% of the total composition.

In yet another embodiment, metal, metal oxide, or salt and acid combined are present in the range of about 50% to about 98% of the total composition and zeolite comprises about 2% to about 50% of the total composition.

In a specific embodiment of the invention, relating to use in a deodorizing composition, e.g. in a cat litter deodorizer, carpet deodorizer or shoe odor deodorizer, the acid comprises about 85-95% of the total composition, the metal oxide, metal or salt comprises about 0.5 to about 5% of the total composition and the synthetic zeolite comprises about 1% to about 12% of the total composition based on the sum of these three components being 100%.

In a more specific embodiment, the odor reducing composition of the invention comprises about 0.5 to about 2.0% metal, metal oxide, or salt, preferably ZnO, about 9 to about 11% zeolite; and about 88 to about 89.5% acid, preferably aspartic acid or fumaric acid.

In yet another specific embodiment, the odor reducing composition of the invention comprises about 11% aspartic acid, about 24.5% adipic acid, about 43% ZnO and about 21.5% zeolite.

In still another specific embodiment, the odor reducing composition of the invention comprises about 90-94% acid, preferably aspartic acid or fumaric acid, about 1-3% metal, metal oxide, or salt, preferably ZnO, and about 5-7% zeolite.

In yet another embodiment, an odor reducing composition of the invention includes a diluent. The amount of diluent in the odor reducing composition of the invention is from 0 to about 70%, i.e. the amount of acid, metal, metal oxide, or salt, and zeolite in the odor reducing composition being about 30 to 100%. Any convenient and compatible diluent, that is generally inert, may be used, including but not limited to sodium sulfate, sodium chloride, sodium bicarbonate, sodium carbonate, clay, sepiolite, palygorskite, activated carbon, activated carbon filter, activated alumina, sepiolite-admixed paper, silica gel, activated clay, vermiculite, diatomaceous earth, pulp, fibers, cloth, polymeric porous body, and a natural zeolite, e.g. a natural, inorganic zeolite such as clinoptilolite. The diluent may be selected based on the undesired odor or application.

In a specific embodiment where a diluent is used, the odor reducing composition of the invention comprises about 30-38% aspartic acid or fumaric acid, about 0.5-1% ZnO, about 1-2% zeolite, and about 60-67% clinoptilolite.

In addition, in an embodiment of the invention for reducing odor, the composition optionally may contain other components if desired, including, but not limited to, biocides, chelants and absorbents, mixtures thereof and the like. Other components that may be optionally incorporated usefully into a composition of this invention for reducing odor are selected in accordance with specific applications of the invention after reading this specification.

In some instances, it may be desirable to combine the above described composition for reducing odor directly with a substance that, in the absence of the odor reducing composition, emits an odor during use. As used herein, the term "odor emitting" means to diffuse or contain a pleasant or unpleasant scent that stimulates the olfactory organ. Accordingly, as used herein, an odor emitting substance is any substance capable of stimulating the olfactory organ, whether offensive or non-offensive. Further, an odor emitting environment is an environment capable of stimulating the olfactory organ, whether offensive or non-offensive non-limiting. Examples of non-offensive odors include perfume and sweet smells non-limiting. Examples of offensive odors include ammonia and sulfurous odors.

The odor reducing composition of the invention may be used alone or may be used in combination with other materials or articles to remove odor. The odor reducing composition of the invention is simply admixed with the materials or articles to remove the odor emitting substances. For example, the odor reducing composition of the invention may be incorporated into personal care articles like foot powders, pads, laundry preparations, pet litters and cleaning products. The composition of the invention may also be used with deodorizers, such as carpet deodorizers and vehicle deodorizers. Often, deodorizers work by masking odors with perfumes and other odors. Prior art odor reducing substances have failed to produce a composition that effectively adsorbs odor without the use of perfuming masks, which are offensive to many. While some in the past may have succeeded to produce compositions that adsorb certain odors, no prior art composition or method has been proven to effectively adsorb the broad spectrum of odors effectively adsorbed by the present invention. For example, any commercially available pet litter may be admixed with an effective amount of the composition of the present invention to adsorb any masking odors that may be irritating or offensive. Also, a commercially available odor controlling substance like carpet deodorizer, may be combined with the present composition to increase the overall effectiveness of its odor adsorption. Accordingly, less of the commercially available odor controlling substances is necessary for effective odor control, making the incorporation of the odor reducing composition of the invention cost effective.

The concentration of the odor reducing composition of the invention is largely dependent upon the specific application of this invention and would be readily apparent to one of ordinary skill in the art. In one embodiment of the invention, the odor reducing composition of the invention is admixed with pet litter to reduce any emitted odor or to improve the odor reducing capabilities of the litter itself. The odor reducing composition of the invention is added to the used or unused pet litter in an amount effective to reduce any undesired odors emitted from the litter thereby producing an odor-controlled pet litter, Any effective amount of the odor reducing composition of the invention may be added to used or unused pet litter or other articles, which in the absence of the present invention emits an odor.

In one embodiment, the odor-controlled pet litter comprises in the range of about 0.1% to about 20% of the odor reducing composition of the invention.

In a preferred embodiment, the odor-controlled pet litter comprises in the range of about 0.1% to about 10% of the odor reducing composition of the invention.

In a more preferred embodiment, the odor-controlled pet litter comprises in the range of about 1% to about 5% of the odor reducing composition of the invention.

In yet another embodiment of this invention, the odor reducing composition of the invention is optionally admixed with one or more effective deodorizers to reduce odors emitted from the deodorizer or to increase the odor reducing capabilities of the deodorizer itself. Where the odor reducing composition of the invention comprises deodorizers or is used in some useful combination with one or more deodorizers.

In another embodiment comprising cat litter deodorizer, carpet and or shoe deodorizer, the invention comprises about 88 to about 89.5% acid, about 9 to about 11% zeolite and about 0.5 to about 2% metal oxide based on the sum of these components being 100%.

In some instances, it may be impractical to directly combine the present compositions with substances that, in the absence of the present invention, emit odor. For example, many personal care articles like menstrual pads and diapers cannot be effectively admixed with the present composition. Therefore, and in one embodiment of the invention, the odor reducing composition of the invention may be simply placed in contact with or incorporated within articles, which in the absence of the odor reducing composition of the invention emit odor during use, to thereby produce odor-controlled articles. The odor reducing composition of the invention may be placed in contact with or incorporated into any article to remove the odor produced during use of that article. For example, the odor reducing composition of the invention may be placed in contact with or incorporated into pads, including diapers, adult incontinence products, and menstrual pads, tissue, lagoons, bandages, dressings, surgical sponges, personal care articles, cleaning products, food tray liners, room and vehicle, e.g. auto, truck, air transport and trains, deodorizers like solid air fresheners, bedding, garbage bags, clothing, shoes and carpet. The present composition may be incorporated within the above articles in any conventional and effective manner known to those of skill in the art.

A method for reducing odor using the above odor reducing composition(s) is also provided according to the present invention. The method for reducing odor includes contacting an effective amount of an odor reducing composition of the invention with at least one article, which, in the absence of the odor reducing composition of the invention, emits an odor during use, and removing the emitted odor from the article. Further, according to the invention, any article that emits odor in the absence of the odor reducing composition of the invention is suitable herein. Unlike prior art odor control compositions, the present composition effectively removes a wide variety of odors including ammonia and sulfurous odors. Without being bound by theory it is believed that the mechanism of odor removal of this invention includes physical and chemical sorption. In an embodiment, the composition of the present invention is admixed with or placed in contact with pads, lagoons, tanks, animal waste, bandages, dressings, surgical sponges, catamenial devices, beef, poultry, and fish tray liners, personal care articles, foot powders, laundry preparations, pet litters such as cat litter and dog litter, cleaning products, deodorizers, bedding, floors, garbage cans, diaper pails, refrigerators, carpet and any substance that emits odor in the absence of the odor reducing composition of the invention. The composition then removes the emitted odor.

In another embodiment, an effective amount of the odor reducing composition of the invention is placed in contact with an odor emitting environment and a sufficient amount of time is allowed to pass for the odor reducing composition to essentially and effectively remove the odor.

In one specific embodiment, the composition of the present invention is enclosed in an article that allows for movement and containment of the composition of this invention. The article may be placed in an odor emitting environment where odor control is desired. For example, the present composition may be placed in a box, bound in a cloth, or bag and placed in a closet, bathroom, or drawer or the like to control odor. The only restriction is that the article which encloses or contains a composition of this invention be sufficiently porous for the odor reducing composition to remove any undesired odor.

An effective amount of the odor reducing composition of the present invention is used in the method for reducing odor of the invention. An effective amount is the amount necessary for the odor reducing composition of the invention present to effectively remove an emitted odor from an odor emitting article or environment. Accordingly, the effective amount used in the present method varies depending on the specific odors emitted, as well as the environment of use among other factors. However, the effective amount of the odor reducing composition of the invention necessary for removing an odor would be readily apparent to one skilled in the art without undue experimentation after reading this specification.

### EXAMPLES

The following examples illustrate specific embodiments of the invention without limiting the scope of the invention in any way.

In the examples which follow, aspartic acid was acquired from Solutia Inc., 385 Marshall Avenue, St. Louis, MO 63119, CBV 901 zeolite catalyst was acquired from Zeolyst International at Valley Forge, PA; Smellrite® was acquired from Universal Oil Products, 307 Fellowship Road, Mount Laurel, IL.

Unless otherwise specified, use of "%" is on a weight basis.

### EXAMPLE 1

Two samples (Samples 1-2) of an odor reducing composition of the invention and a comparative sample were prepared by adding the following to three individual jars, stirring the contents of the jars, capping the jar and shaking the jars.

| | |
|---|---|
| Sample-1 | 100 g zinc oxide; |
| | 100 g L-aspartic; and |
| | 100 g zeolite (CBV 901). |
| | |
| Sample-2 | 100 g zinc oxide; |
| | 100 g L-aspartic; and |
| | 100 g Smellrite®. |
| | |
| *Sample-3 | 100 g zinc oxide; |
| | 100 g L-aspartic; and |
| | 100 g zeolite (UOP PA 451). |

| | |
|---|---|
| * comparative sample | |

These two samples (Samples 1-2) were then tested against comparative samples of unused Arm & Hammer@ cat litter deodorizer and Sample-3 by placing three teaspoons of each of the samples and the unused Arm & Hammer@ cat litter deodorizer in a jar containing used pet litter. The results demonstrate that samples containing Smellrite® or Zeolyst™ CBV 901 are preferred over samples containing PA 451. The results are summarized in Table 1 below.

**Table 1**

| Sample Composition | Amount added to pet litter | Efficacy |
|---|---|---|
| Sample-1 | 3 teaspoons | No odor |
| Sample-2 | 3 teaspoons | No odor |
| Sample-3* | 3 teaspoons | Odor |
| Arm & Hammer@ cat litter deodorizer* | 3 teaspoons | Masks odor with fragrance effectively |
| 1/3 Sample-1 + 2/3 Sample-3 | 3 teaspoons | weak odor |
| 1/3 Sample-2 + 2/3 Sample-3 | 3 teaspoons | very weak odor |

| | | |
|---|---|---|
| *comparative sample | | |

The results also indicate that compositions of this invention utilizing Srriellrite® and Zeolyst™ CBV 901 were demonstrated to be very effective in removing odors caused by solid cat litter, while zeolite UOP PA 451 (a comparative composition) was less effective.

### EXAMPLE 2

### ODOR CONTROL TESTING OF AMMONIA

The following (odor reducing) compositions illustrative of this invention were prepared for odor control testing. Specifically, the ability to remove ammonia was tested for various odor reducing compositions. Samples were prepared by following the method of Example 1. 29.5% NH₄OH was added to each of the following odor reducing compositions. 2 or 3 drops of 29.5% NH₄OH (10 drops = 0.28 g) were added to about 10 g of the odor reducing compositions in a 2 oz. jar. The 29.5% NH₄OH and the odor reducing compositions were admixed and allowed to set for about 10 minutes with the lid tightly closed. If there was no odor upon removal of the lid, as determined by smell, additional 29.5% NH₄OH was added and the test was repeated. As used herein, the grams of 29.5% NH₄OH adsorbed represents the maximum amount of 29.5% NH₄OH removed by 10 grams of the respective samples. Accordingly, at any concentration level above the amounts indicated, an ammonia odor was emitted from the mixture. The results are summarized in Tables 2 and 3.

The above results and those in Tables 2 and 3 below indicate that both L-aspartic and adipic acid are demonstrated to be very effective in removing odors caused by ammonia.

**Table 2**

| Odor Reducing Composition Number | L-aspartic acid | zinc oxide | Zeolyst™ CBV 901 | Advera® 401 | 13X powder (diluent zeolite) | Grams of 29.5% NH₄OH** in a 10g sample |
|---|---|---|---|---|---|---|
| 1 | 33 g | 33 g | 33 g | | | 0.76 g |
| 2 | 33 g | 33 g | 3 g | 30 g | | 1.12 g |
| 3 | 33 g | 33 g | 3 g | | 30 g | 0.50 g |
| 4 | 33 g | 33 g | 11 g | 22 g | | 1.96 g |
| 5 | 33g | 33g | 11 g | | 22g | 0.36g |
| 6 | 33 g | 33 g | 6 g | 27 g | | 1.54 g |
| 7 | 33 g | 33 g | 6 g | | 27g | 0.45 g |
| 8 | 40 g | 40 g | 20 g | | | 1.90 g |
| 9 | 33 g | 44 g | 22 | | | 2.10 g |
| *10 | 50 g | 50 g | | | | 1.48 g |

| | | | | | | |
|---|---|---|---|---|---|---|
| *comparative sample | | | | | | |
| **amount of 29.5% NH₄OH added before persistent odor of ammonia is detectable | | | | | | |

**Table 3**

| Odor Reducing Composition Number | Adipic acid | Zinc Oxide | Zeolyst™ CBV 901 | Advera® 401 | 13X powder (diluent zeolite) | Grams of 29.5% NH₄OH**in a 10g sample |
|---|---|---|---|---|---|---|
| 1 | 33 g | 33 g | 33 g | | | 2.10 g |
| 2 | 33 g | 33 g | 3 g | 30 g | | 1.68 g |
| 3 | 33 g | 33 g | 3 g | | 30 g | 0.56g |
| 4 | 33 g | 33 g | 11 g | 22 g | | 2.10 g |
| 5 | 33 g | 33 g | 11 g | | 22 g | 0.42 g |
| 6 | 33 g | 33 g | 6 g | 27 g | | 2.10 g |
| 7 | 33 g | 33 g | 6 g | | 27 g | 0.70 g |
| 8 | 40g | 40g | 20g | | | 2.24g |
| 9 | 33 g | 44 g | 22 g | | | 2.24 g |
| *10 | 50 g | 50 g | | | | 2.10 g |

| | | | | | | |
|---|---|---|---|---|---|---|
| *comparative sample | | | | | | |
| **Amount of 29.5% NH₄OH added before persistent odor of ammonia is detectable. | | | | | | |

### EXAMPLE 3

### ODOR CONTROL TESTING OF AMMONIA USING ODOR REDUCING COMPOSITIONS OF THE PRESENT INVENTION

3.3 g of each acid indicated in Table 8 (below) were admixed with 3.3 g zinc oxide, available from Fisher Scientific and 3.3 g Advera® 401 (PQ Corporation) to form an odor reducing composition in accordance with the present invention. About 10 g of the respective odor reducing compositions were placed into individual 2 oz. jars. 29.5 % NH₄OH was added to the odor reducing compositions, capped and allowed to set for 10 minutes. If there was no odor, detected qualitatively after ten minutes elapsed, more 29.5% NH₄OH was added and the test was repeated until an odor was emitted. The results of this Example are summarized in Table 4 below and demonstrate that odors caused by ammonia can be removed by the action of odor reducing compositions of the inventions that include these acids.

**Table 4**

| Acid used in the odor reducing composition | Drops/grams 29.5% NH₄OH** |
|---|---|
| *Ascorbic acid | 70 = 1.96 g |
| *Citric acid | 70 = 1.96 g |
| Fumaric acid | 120 = 3.36 g |
| Glutaric acid | 85 = 2.38 g |
| Methyl Succinic acid | 90 = 2.52 g |
| *Malic acid | 40 = 1.12 g |
| o-Phthalic acid | 85 = 2.38 g |
| Succinic acid | 90 = 2.52 g |
| tartaric acid | 90 = 2.52 g |

| | |
|---|---|
| *comparative samples | |
| **Amount of 29.5% NH₄OH added before persistent odor of ammonia is detectable. | |

### EXAMPLE 4

### COMPARATIVE ODOR CONTROL TESTING OF AMMONIA USING ODOR REDUCING COMPOSITIONS OF THE PRESENT INVENTION AND COMMERCIALLY AVAILABLE ZEOLITES ALONE

Samples were prepared following the procedure of Example 1 above. 29.5% NH₄OH was added dropwise (10 drops = 0.28 g) to 10 g of each of the odor reducing compositions, or zeolites shown in Table 5 in a 2 oz jar. Covered with a lid, the jar is shaken and allowed to set for 10 min. If there is no odor, additional 29.5% NH₄OH is added and the test is repeated until odor is emitted. The results of this Example are summarized in Table 5.

**Table 5**

| Odor Reducing Composition / Zeolite | Grams of 29.5% NH₄OH** |
|---|---|
| 33.4% L-aspartic acid | 1.54 g |
| 33.3% zinc oxide | |
| 33.3% Zeolyst™ CBV 901 | |
| 30% Fumaric acid | 3.22 g |
| 10% Sorbic acid | |
| 30% Zinc Sulfate | |
| 30% Smellrite® | |
| 20% L-aspartic acid | 2.24 g |
| 20% Fumaric acid | |
| 20% Zinc Oxide | |
| 30% Advera® 401 | |
| 10% Smellrite® | |
| 20% L-aspartic acid | 1.68 g |
| 20% Fumaric acid | |
| 5% Zinc Sulfate | |
| 15% Copper Sulfate | |
| 30% Advera® 401 | |
| 10% Smellrite® | |
| * Zeolyst™ CBV 901 | 0.14 g |
| * Smellrite® | 0.56 g |

| | |
|---|---|
| * control sample | |
| **Amount of 29.5% NH₄OH added before persistent odor of ammonia is detectable. | |

The results of this Example show the ability of the odor control compositions in accordance with the present invention to remove significantly more ammonia than the use of zeolites alone.

### EXAMPLE 5

### COMPARATIVE ODOR CONTROL TESTING OF AMMONIA USING ODOR REDUCING COMPOSITIONS OF THE PRESENT INVENTION AND OTHER COMMERCIALLY AVAILABLE ODOR CONTROL COMPOSITIONS

10 g of each of the odor control compositions listed below were placed into a 2 oz jar. 29.5% NH₄OH, from Fisher Scientific, was added to each jar, the jar was shaken and allowed to set for about 10 min. The odor was monitored. If there was no odor after ten minutes had elapsed, more 29.5% NH₄OH was added and the test repeated. The results of this Example are summarized in Table 6.

**Table 6**

| Odor Control Composition | 29.5% NH₄OH** |
|---|---|
| *Tidy Cat@ Scoop (Ralston Purina Company) | 0.06 g |
| *Super Scoop™ (Arm & Hammer®) | 0.28 g |
| *Ammo Cat@ (Aquarium Pharm.) | 0.28 g |
| *Pearl Fresh (PETsMART) | 0.56 g |
| *Odor Pro (Odor Pro) | 0.28 g |
| *Odor Sentry (Ammonia Hold) | 0.14 g |
| *Odorz Out (No Stink) | 0.28 g |
| *Tidy Cat® cat box deodorizer (Ralston Purina Company) | Very Strong Odor <0.14 g |
| *Cat litter deodorizer (Arm & Hammer®) | Strong odor <0.14 g |
| 60% L-aspartic; | 1.54 g |
| 10% Smellrite®; and | |
| 30% zinc oxide. | |

| | |
|---|---|
| *comparative example | |
| **Amount of 29.5% NH₄OH added before persistent odor of ammonia is detectable. | |

The results of this Example indicate that the compositions for reducing odor of the present invention remove substantially more ammonia odor than other commercially available odor control compositions.

### EXAMPLE 6

### COMPOSITION FOR REDUCING ODOR OF THE PRESENT INVENTION USED IN COMBINATION WITH PET LITTER

60 g aspartic acid, 10 g Smellrite® and 30 g zinc oxide were admixed to produce a composition for reducing odor in accordance with the present invention. A comparative sample of Tidy Cat® scoop, commercially available from Ralston Purina Company, St. Louis, MO, was combined with the composition in a 2 oz. jar at the amounts indicated in the Table 7 below to form four distinct odor reducing compositions in accordance with the present invention. 29.5% NH₄OH, available from Fisher Scientific, was added to each of the final compositions dropwise until an ammonia odor was emitted. If no odor was emitted additional 29.5% NH₄OH was added and the test was repeated until an odor was emitted. The results of this Example are summarized in Table 7.

**Table 7**

| Tidy Cat® scoop | Odor Reducing Composition of the Present Invention | 29.5% NH₄OH** |
|---|---|---|
| 10 g* | 0 g | 0.08 g |
| 9 g | 1 g | 0.42 g |
| 8 g | 2 g | 0.84 g |
| 7 g | 3 g | 0.98 g |
| 0 | 10 g | 1.54 g |

| | | |
|---|---|---|
| *control sample | | |
| ** Amount of 29.5% NH₄OH added before persistent odor of ammonia is detectable. | | |

The results of this Example indicate that the odor reducing composition of the present invention adsorbed substantially more ammonia odor than Tidy Cat® Scoop alone. The results also indicated that the odor reducing compositions formed by the addition of the composition of the present invention to other pet litters resulted in enhanced adsorption capabilities as compared to the use of the pet litter alone.

### EXAMPLE 7

### COMPARATIVE ODOR CONTROL TESTING OF SULFIDE

A 0.2% aqueous sulfide solution was prepared by mixing sodium sulfide, acetic acid and water. An odor reducing composition of the present invention was prepared by mixing 60 g L-aspartic acid, 10 g Smellrite® and 30 g zinc oxide. 0.1 gram of the above odor reducing composition was placed in a 2 oz jar and a lid was placed on the jar. One gram of the other commercially available odor control compositions were placed in individual 2 oz. jars. The sulfide solution was added to each of the jars dropwise and admixed with the respective compositions. If no sulfide was detected after approximately 10 minutes, additional sodium sulfide was added and the test was repeated until sulfide was detected. The results are summarized in Table 8 below.

**Table 8**

| Sample | Drops of 0.2% sodium sulfide solution |
|---|---|
| Odor Reducing Composition | 140-250 |
| *Tidy Cat® scoop (Ralston Purina) | 3 |
| *Super Scoop™ (Arm & Hammer) | 2 |
| *Ammo Cat® (Aquarium Pharmaceuticals, Inc., Chalfont, PA) | 1 |
| *ExquisiCat® Pearl Fresh cat litter(PETsMART, Inc. Phoenix, AZ) | 2 |
| *Odor Pro (Odor Pro®) | 2 |
| *Odor Sentry ™ pet odor eliminator (Ammonia Hold, Inc.@, Little Rock, AR) | 2 |
| *Odorz Out (No Stink) | 8 |
| *Tidy Cat box deodorizer (Ralston Purina) | 1 |
| *Cat Litter deodorizer (Arm & Hammer) | 1 |

| | |
|---|---|
| *comparative examples | |

This Example shows that the odor reducing formulation is at least 15 times as effective as commercially available products available for the removal of sulfide odors.

### EXAMPLE 8

### EFFECT OF ZINC CONCENTRATION

A 0.2% aqueous sulfide solution was prepared by combining sodium sulfide, acetic acid and water. Compositions for reducing odor were prepared in accordance with the present invention by mixing L-aspartic acid, zinc oxide, Smellrite® and CBV 901 in the proportions indicated below in Table 9. 0.1 g of the resulting compositions were each placed in a jar. The sulfide solution was added dropwise to each of the jars until an odor was emitted. If no odor was emitted after approximately ten minutes, additional sulfide solution was added, and the test was repeated until an odor was emitted. Separately, in different containers, 29.5% NH₄OH was added to each 10g sample until and odor of ammonia was detected. The results of this Example are summarized in Table 9 below.

**Table 9**

| % L-aspartic | % zinc Oxide | % Smellrite® | % Zeolyst™ CBV 901 | Drops Sulfide Solution | Drops 29.5% NH₄OH** |
|---|---|---|---|---|---|
| 60 | 2 | 10 | 28 | 15 | 95 |
| 60 | 5 | 10 | 25 | 25 | 90 |
| 60 | 10 | 10 | 20 | 60 | 80 |
| 60 | 20 | 10 | 10 | 125 | 65 |
| 60 | 30 | 10 | 0 | 225 | 55 |

| | | | | | |
|---|---|---|---|---|---|
| **Amount of 29.5% NH₄OH added before persistent odor of ammonia is detectable. | | | | | |

The results demonstrate that these formulations are effective in controlling both ammonia and sulfide odors over a broad range of zeolite and metal oxide concentrations.

### EXAMPLE 9

### ODOR CONTROL TESTING OF SULFUR USING COMPOSITIONS FOR REDUCING ODOR COMPRISING LOW CONCENTRATIONS OF METAL OXIDE

Zinc oxide, Smellrite® and L-aspartic acid were admixed to form a composition for reducing odor in accordance with the invention having the following approximate composition: 0.5% zinc oxide, 10% Smellrite® and 89.5% L-aspartic. Lead acetate strips were used to detect the presence of sulfide after 10 minutes. A 0.2% aqueous sulfide solution was prepared by mixing sodium sulfide and acetic acid in water. The sulfide solution was added dropwise to 1 g of the composition. If an odor was not emitted, additional sulfide solution was added until odor was emitted. 14 drops of sulfide solution was added before an odor was emitted.

Zinc oxide, Smellrite® and L-aspartic acid were admixed to form a composition for reducing odor in accordance with the invention having the following approximate composition: 0.5% zinc oxide, 10% Smellrite®, 59.5% Clinoptilolite and 30% L-aspartic acid were admixed to form a composition for reducing odor in accordance with the invention. A 0.2% aqueous sulfide solution was prepared by mixing sodium sulfide, acetic acid and water. The sulfide solution was added dropwise to 1 g of the composition. If an odor was not emitted, additional sulfide solution was added until an odor was emitted. 10 drops of sulfide solution was added before an odor was emitted.

Zinc oxide, Smellrite® and L-aspartic acid were admixed to form a composition for reducing odor in accordance with the invention having the following approximate composition: 1.0% zinc oxide, 10% Smellrite® and 89% L-aspartic acid. A 0.2% aqueous sulfide solution was prepared by mixing sodium sulfide, acetic acid and water. The sulfide solution was added dropwise to 1 g of the composition until an odor was emitted. 24 drops of sulfide solution was adsorbed before an odor was emitted.

Zinc oxide, Smellrite® and L-aspartic acid were admixed to form a composition for reducing odor in accordance with the invention having the following approximate composition: 2.0% zinc oxide, 10% Smellrite® and 88% L-aspartic were admixed to form a composition for reducing odor in accordance with the invention. A 0.2% aqueous sulfide solution was prepared by mixing sodium sulfide, acetic acid and water. The sulfide solution was added dropwise to 1 g of the composition until an odor is emitted. 140 drops of sulfide solution was adsorbed before an odor was emitted.

These results indicate the preferred range of zinc oxide in an odor reducing composition of this invention for use in an application where sulfurous odors are emitted.

### EXAMPLE 10 - COMPARATIVE

### ODOR CONTROL TESTING OF B-CYCLODEXTRIN HYDRATE AND FEBREZE®

0.5 g B-cyclodextrin hydrate was placed in a 2 oz jar with 2 drops of 0.2% aqueous sulfide solution and a lead acetate strip. The lead acetate strip turned black indicating sulfide present in the air.

5.0 g of B-cyclodextrin hydrate was placed in a 2 oz jar with 2 drops of 29.5% NH₄OH. A strong odor of ammonia was emitted.

1.0 g of liquid Febreze® (Proctor & Gamble) was placed in a 2 oz jar with 1 drop of a 0.2% aqueous sulfide solution and a lead acetate strip. The lead acetate strip turned black.

10.0 g of liquid Febreze® was placed in a 2 oz jar with 5 drops of 29.5% NH₄OH. A strong odor was detected.

The results of this example demonstrate that neither B-cyclodextrin hydrate nor Febreze® effectively remove ammonia or sulfide odors.

### EXAMPLE 11- COMPARATIVE

### CAT LITTER ODOR CONTROL

10 g of each of the samples listed Table 11 below were placed in individual 2 oz. jars. 29.5% NH₄OH was added dropwise to the jar and admixed until an odor was emitted.

1 g of each of the samples listed in the Table 11 were placed in individual 2 oz. jars. A 0.2% aqueous sulfide solution was added dropwise to each of the samples until an odor was detected. The results are summarized in Table 11 below.

**Table 11**

| Samples | Drops Ammonia adsorbed | Drops Sulfur adsorbed |
|---|---|---|
| *Arm & Hammer® Carpet & Room Deodorizer | < 10 drops | < 3 drops |
| *Arm & Hammer® Cat Litter Deodorizer | < 10 drops | < 3 drops |
| *Simple Solution Carpet & Room Deodorizer | < 10 drops | < 3 drops |
| *Glade Pet Odors Carpet & Room Deodorizer | < 10 drops | < 3 drops |
| *Stop Litter Odor | < 10 drops | < 3 drops |
| *Pet Clear All Natural | < 10 drops | < 3 drops |
| *Captain Cat@ Cat Litter Deodorizer | < 10 drops | < 3 drops |

| | | |
|---|---|---|
| *comparative example | | |

The results indicate that the odor reducing composition of the invention is more effective at removing both ammonia and sulfide odors than these commercially available compositions for odor reduction.

### EXAMPLE 12

The effectiveness of odor reducing compositions of the invention in controlling the odors of 3-methylindole, sodium methoxide, methylsalicylate, triethylamine, isovaleric acid, and dimethylsulfide was compared against the effectiveness of the individual components of the compositions and baking soda, i.e. sodium bicarbonate.

Two odor reducing compositions of the invention were tested. The first odor reducing composition of the invention was 89% L-aspartic acid, 1% ZnO, and 10% Smellrite® zeolite (Invention #1). The second odor reducing composition of the invention was 88% L-aspartic acid, 2% ZnO, and 10% Smellrite® zeolite (Invention #2). The odor emitting compounds were tested neat, and triethylamine (0.1%), dimethylsulfide (0.1%), and isovaleric acid (0.82%) were also tested as solutions in water. All odor emitting compounds except methylsalicylate were obtained from Aldrich Chemical Company. Methylsalicylate was obtained from Sigma Chemical Company.

The amount of neat odor emitting compounds added to each serum bottle is given in Table 12. In the tests using the water solutions of the odor emitting compounds, 500 µL of the solutions were used for all samples.

**Table 12**

| Sample | 3-methylindole (mg) | Sodium methoxide (mg) | Methylsalicylate (mg) | Triethylamine (mg) | Isovaleric acid (mg) | Dimethylsulfide (mg) |
|---|---|---|---|---|---|---|
| Control - as is | 18 | 22 | 23 | 22 | 23 | 17 |
| Invention #1 | 20 | 29 | 25 | 19 | 22 | 28 |
| Smellrite | 21 | 21 | 26 | 20 | 21 | 22 |
| L-aspartic acid | 22 | 19 | 25 | 24 | 23 | 28 |
| Zinc oxide | 35 | 21 | 28 | 25 | 21 | 21 |
| Baking soda | 30 | 19 | 19 | 19 | 21 | 19 |

The amount of the samples used in the tests were as follows: Invention #1 (1.0g), Invention #2 (1.0g), Smellrite® (0.1 g), L-aspartic acid (0.89g for Invention #1 and 0.88g for Invention #2), zinc oxide (0.01g for Invention #1 and 0.02g for Invention #2), and baking soda (1.0 g).

Samples were prepared using 20 mL serum bottles with septum caps, and gas tight syringes were used to sample the headspace in the serum bottles. The sample was weighed into the serum bottle, the odor emitting compounds were added, and the sample bottles capped. The sample and the odor emitting compound in the serum bottle was mixed in each case. The serum bottle was then allowed to stand for 30 minutes and a 100 µL sample was taken from headspace of the serum bottle using a gas tight syringe and then injected into a gas chromatograph (GC) for analysis.

The GC analysis was performed using a H-P 5890-II GC with a FID detector and a Split/Splitless injector, and a H-P 3396 integrator. The column used was a capillary H-P Ultra-2 (50m x 0.32mm x 0.52µm). The GC conditions used were: injector temperature = 300°C, detector temperature = 330°C, oven temperature program = 70°C initial hold for 4 min., raise temperature 7°C/min. to 235°C, hold at 235°C for 4 min. with total run length of 31.6 min. The split was turned off at injection and turned on after 30 seconds.

The GC results are presented in Tables 13 and 15 (area counts in headspace) and Tables 14 and 16 (% Odor). % Odor was expressed as % of control ("as-is") and calculated as follows: (area sample/area control) x 100. Complete removal of odor is represented as 0% and the control is 100%.

**Table 13**

| GC Analysis - Area Counts in Headspace | | | | | | |
|---|---|---|---|---|---|---|
| Sample | 3methylindole | Sodium methoxide | Methylsalicylate | Triethylamine | Isovaleric Acid | Dimethylsulfide |
| Control - as is | 4000 | 20378 | 38790 | 8590381 | 53990 | 10670136 |
| Invention #1 | 0 | 0 | 2049 | 7591005 | 20048 | 8803891 |
| Smellrite | 1226 | 0 | 15397 | 5754746 | 46745 | 14319488 |
| L-aspartic acid | 3580 | 19129 | 33871 | 6236176 | 47685 | 4056472 |
| Zinc oxide | 2706 | 38097 | 32463 | 7113763 | 48121 | 7087306 |
| Baking soda | 2669 | 16261 | 38721 | 3019376 | 2594 | 12928528 |

**Table 14**

| % Odor | | | | | | |
|---|---|---|---|---|---|---|
| Sample | 3-methylindole | Sodium methoxide | Methylsalicylate | Triethylamine | Isovaleric Acid | Dimethylsulfide |
| Control - as is | 100% | 100% | 100% | 100% | 100% | 100% |
| Invention #1 | 0% | 0% | 5.3% | 88.4% | 37.1% | 82.5% |
| Smellrite | 30.7% | 0% | 39.7% | 67.% | 86.6% | 134.2% |
| L-aspartic acid | 89.5% | 93.9% | 87.3% | 72.6% | 88.3% | 38.% |
| Zinc oxide | 67.7% | 187.% | 83.7% | 82.8% | 89.1% | 66.4% |
| Baking soda | 66.7% | 79.8% | 99.8% | 35.2% | 4.8% | 121.2% |

**Table 15**

| GC Analysis - Area Counts in Headspace | | | |
|---|---|---|---|
| Sample | Triethylamine (0.1% in water) | Isovaleric acid (0.82% in water) | Dimethylsulfide (0.1% in water) |
| Control - as is | 23500 | 3655 | 153633 |
| Invention #2 | 0 | 485 | 2459 |
| Smellrite | 0 | 0 | 1426 |
| L-aspartic acid | 0 | 509 | 126284 |
| Zinc oxide | 18640 | 0 | 148158 |
| Baking soda | 4473 | 319 | 143740 |

**Table 16**

| % Odor | | | |
|---|---|---|---|
| Sample | Triethylamine (0.1% in water) | Isovaleric acid (0.82% in water) | Dimethylsulfide (0.1% in water) |
| Control - as is | 100% | 100% | 100% |
| Invention #2 | 0% | 13.3% | 1.6% |
| Smellrite | 0% | 0% | 0.9% |
| L-aspartic acid | 0% | 13.9% | 82.2% |
| Zinc oxide | 79.3% | 0% | 96.4% |
| Baking soda | 19.% | 8.7% | 93.6% |

The results demonstrate that the odor reducing compositions of the invention (Invention #1 and Invention #2) reduced the amount of odor for each odor emitting compound compared to the control samples. In addition, the Invention #1 sample demonstrated improved performance over the performance of the individual components for odor emitting compounds 3-methylindole, methylsalicylate, and isovaleric acid. The Invention #1 sample also demonstrated equivalent overall effectiveness to Smellrite® zeolite for sodium methoxide. It is believed that the neat samples of triethylamine and dimethylsulfide were used at too high a loading, i.e. the amount of odor causing material greatly exceeded the capacity of the odor reducing materials being tested.

Therefore, the data clearly demonstrate that the odor reducing compositions of the invention are effective for a wide range of odor emitting compounds.

## Claims

1. A composition for reducing odor comprising:
(a) 0.1 wt. % to 99.7 wt. % of at least one acid having a pKₐ greater than 2.9 and less than 6, water solubility less than 45 g/100g H₂O, and an oral rat LD₅₀ greater than 2200 mg/kg;
(b) 0.1 wt. % to 99.7 wt. % of at least one synthetic zeolite having at least 90 percent of its tetrahedral oxide units as SiO₂ tetrahedra, a capacity for adsorbed water of not greater than 10 weight percent when measured at 25 °C and water vapor pressure at 4.6 torr, and pore apertures at least 5.5 Å in diameter, wherein the original water of hydration has been substantially removed; and
(c) 0.1 wt. % to 99.7 wt. % of a substance selected from the group consisting of a metal, metal oxide, and any combination thereof;
wherein the sum of (a), (b), and (c) is 100 wt. %.

2. The composition of claim 1 wherein said acid is at least one acid selected from the group consisting of an organic acid, amino acid and any combination thereof.

3. The composition of claim 1 wherein said metal oxide is selected from the group consisting of zinc oxide, copper oxide, iron oxide, manganese oxide, tin oxide and silver oxide.

4. The composition of claim 1 wherein said metal is selected from the group consisting of zinc, copper, iron, manganese, tin and silver.

5. The composition of claim 1 further comprising an article or substance that, in the absence of said composition for reducing odor, emits an odor during use.

6. The composition of claim 5 wherein said article or substance is at least one selected from the group consisting of personal care articles, foot powders, laundry preparations, pet litters, cleaning products and deodorizers.

7. The composition of claim 1 having about (i) 33 to about 99% acid, (ii) about 0.5 to about 40% metal or metal oxide, and (iii) about 5 to about 50% synthetic zeolite, wherein the sum of (i), (ii) and (iii) is 100%.

8. The composition of claim 7 having about 88 to about 89.5% acid, about 0.5 to about 2% metal or metal oxide and about 9 to about 11% synthetic zeolite.

9. The composition of claim 7 having about 90 to about 94% acid, about 1 to about 3% metal or metal oxide and about 5 to about 7% synthetic zeolite.

10. The composition of claim 1 wherein said acid and said metal oxide or metal combined comprise in the range of about 24% to about 99.7% of the total composition and said zeolite comprises in the range of about 0.3% to about 76% of the total composition.

11. The composition of claim 10 wherein said acid and said metal oxide or metal combined comprise in the range of about 50% to about 98% of the total composition and said zeolite comprises in the range of about 2% to about 50% of the total composition.

12. The composition of claim 1 further comprising a diluent.

13. The composition of claim 12 wherein said diluent is sodium bicarbonate or a natural zeolite.

14. The composition of claim 13 wherein said diluent is clinoptilolite.

15. The composition of claim 14 having about 30-38% aspartic acid or fumaric acid, about 0.5-1% ZnO, about 1-2% zeolite, and about 60-67% clinoptilolite.

16. An odor-controlled article comprising:
an effective amount of an odor reducing composition according to any one of claims 1 to 4 or 7 to 15; and
an article that, in the absence of said odor reducing composition, emits odor
during use, in contact with said odor reducing composition .

17. The article of claim 16 wherein said odor emitting article is selected from the group consisting of pads, tissue, lagoons, bandages, dressings, surgical sponges, personal care articles, cleaning products, room deodorizers, vehicle deodorizers, and garbage bags.

18. A method for reducing odor that comprises contacting an effective amount of an odor reducing composition according to any one of claims 1 to 4 or 7 to 15, with an article that, in the absence of said odor reducing composition, emits an odor during use, for a sufficient time to effectively remove said odor; and removing said emitted odor from said odor emitting article.

19. The method of claim 18 wherein said odor emitting article is selected from the group consisting of pads, lagoons, tanks, animal waste, bandages, dressings, surgical sponges, catamenial devices, beef, poultry and fish trays, personal care articles, foot powders, laundry preparations, pet litters, cleaning products, deodorizers, bedding, floors, garbage cans, diaper pails, refrigerators, vehicles, and carpet.

20. A method for removing odor from an odor emitting environment comprising: contacting an effective amount of an odor reducing composition according to claim 1 with said odor emitting environment; and allowing a sufficient time to pass for said composition to remove the odor.

21. The method of claim 20 wherein said step of contacting an effective amount of an odor reducing composition with said odor emitting environment comprises contacting an odor reducing composition, wherein said composition is contained within an article that allows for containment of said composition with said odor emitting environment.

22. A composition for reducing odor comprising:
(a) 0.1 wt. % to 99.7 wt. % of at least one acid selected from adipic, aspartic, cyclohexane-1:1-dicarboxylic, cystine, dimethylmalonic, fumaric, sorbic, glutaric, metbylsuccinic, itaconic, or tartaric acid;
(b) 0.1 wt. % to 99.7 wt. % of at least one synthetic zeolite having at least 90 percent of its tetrahedral oxide units as SiO₂ tetrahedra, a capacity for adsorbed water of not greater than 10 weight percent when measured at 25 °C and water vapor pressure at 4.6 torr, and pore apertures at least 5.5 Å in diameter, wherein the original water of hydration has been substantially removed; and
(c) 0.1 wt. % to 99.7 wt. % of a substance selected from the group consisting of a metal, metal oxide, a salt of a metal or metal oxide, and any combination thereof;
wherein the sum of (a), (b), and (c) is 100 wt. %.

23. The composition of claim 22 wherein said metal oxide is selected from the group consisting of zinc oxide, copper oxide, iron oxide, manganese oxide, tin oxide and silver oxide.

24. The composition of claim 22 wherein said metal is selected from the group consisting of zinc, copper, iron, manganese, tin and silver.

25. The composition of claim 22 wherein said salt is selected from the group consisting of a salt of zinc, copper, iron, manganese, tin and silver.

26. The composition of claim 22 having about (i) 33 to about 99% acid, (ii) about 0.5 to about 40% metal, metal oxide or salt of a metal or metal oxide, and (iii) about 5 to about 50% synthetic zeolite, wherein the sum of (i), (ii) and (iii) is 100%.

27. The composition of claim 26 having about 88 to about 89.5% acid, about 0.5 to about 2% metal, metal oxide or salt of a metal or metal oxide and about 9 to about 11% synthetic zeolite.

28. The composition of claim 26 having about 90 to about 94% acid, about 1 to about 3% metal, metal oxide or salt of a metal or metal oxide and about 5 to about 7% synthetic zeolite.

29. The composition of claim 22 wherein said acid and said metal oxide, metal, or salt of metal or metal oxide combined comprise in the range of about 24% to about 99.7% of the total composition and said zeolite comprises in the range of about 0.3% to about 76% of the total composition.

30. The composition of claim 29 wherein said acid and said metal oxide, metal, or salt of metal or metal oxide combined comprise in the range of about 50% to about 98% of the total composition and said zeolite comprises in the range of about 2% to about 50% of the total composition.

31. The composition of claim 22 further comprising a diluent.

32. The composition of claim 31 wherein said diluent is sodium bicarbonate or a natural zeolite.

33. The composition of claim 32 wherein said diluent is clinoptilolite.

34. The composition of claim 33 having about 30-38% aspartic acid or fumaric acid, about 0.5-1 % ZnO, about 1-2% zeolite, and about 60-67% clinoptilolite.

35. The composition of claim 22 wberein said at least one acid is selected from aspartic, fumaric, sorbic, tartaric or adipic acid.

36. The composition of claim 35 wherein said metal oxide or salt of a nutal is selected from the group consisting of zinc oxide and zinc sulfate.

37. An odor-controlled article comprising:
an effective amount of an odor reducing composition according to any one of claims 22 to 34 or 36 and
an article that, in the absence of said odor reducing composition; emits odor during use, in contact with said odor reducing composition.

38. The article of claim 37 wherein said odor emitting article is selected from the group consisting of pads, tissue, lagoons, bandages, dressings, surgical sponges, personal care articles, cleaning products, room deodorizers, vehicle deodorizers, and garbage bags.

39. A method for reducing odor comprising contacting an effective amount of an odor reducing composition according to any one of claims 22 to 34
with an article that, in the absence of said odor reducing composition, emits an odor during use, for a sufficient time to effectively remove said odor; and removing said emitted odor from said odor emitting article.

40. The method of claim 39 wherein said odor emitting article is selected from the group consisting of pads, lagoons, tanks, animal waste, bandages, dressings, surgical sponges, catamenial devices, beef, poultry and fish trays, personal care articles, foot powders, laundry preparations, pet litters, cleaning products, deodorizers, bedding, floors, garbage cans, diaper pails, refrigerators, vehicles, and carpet.

41. A method for removing odor from an odor emitting environment comprising: contacting an effective amount of an odor reducing composition, according to claim 22
with said odor emitting environment; and allowing a sufficient time to pass for said composition to remove the odor.

42. The method of claim 41 wherein said step of contacting an effective amount of an odor reducing composition with said odor emitting environment comprises contacting an odor reducing composition, wherein said composition is contained within an article that allows for containment of said composition with said odor emitting environment.

## Patentansprüche

1. Zusammensetzung zur Verminderung von Gerüchen, umfassend:
(a) 0,1 Gew.-% bis 99,7 Gew.-% von mindestens einer Säure mit einem pKₐ-Wert größer als 2,9 und kleiner als 6, einer Wasserlöslichkeit von weniger als 45 g/100 g H₂O, und einer oralen LD₅₀ bei Ratten von größer als 2200 mg/kg;
(b) 0,1 Gew.-% bis 99,7 Gew.-% von mindestens einem synthetischen Zeolith, bei dem mindestens 90% der tetraedrischen Oxideinheiten SiO₂-Tetraeder sind, mit einer Kapazität für adsorbiertes Wasser von nicht mehr als 10 Gew.-%, gemessen bei 25°C und einem Wasserdampfdruck von 4,6 Torr, und mit einer Porenöffnung von mindestens 5,5 Å Durchmesser, wobei das ursprüngliche Hydratwasser im Wesentlichen entfernt worden ist; und
(c) 0,1 Gew.-% bis 99,7 Gew.-% einer Substanz, ausgewählt aus der Gruppe bestehend aus einem Metall, Metalloxid und jeder Kombination davon;
wobei die Summe von (a), (b) und (c) 100 Gew.-% beträgt.

2. Zusammensetzung nach Anspruch 1, wobei die Säure mindestens eine Säure, ausgewählt aus der Gruppe bestehend aus einer organischen Säure, Aminosäure und jeder Kombination davon, ist.

3. Zusammensetzung nach Anspruch 1, wobei das Metalloxid ausgewählt ist aus der Gruppe bestehend aus Zinkoxid, Kupferoxid, Eisenoxid, Manganoxid, Zinnoxid und Silberoxid.

4. Zusammensetzung nach Anspruch 1, wobei das Metall ausgewählt ist aus der Gruppe bestehend aus Zink, Kupfer, Eisen, Mangan, Zinn und Silber.

5. Zusammensetzung nach Anspruch 1, des Weiteren umfassend einen Gegenstand oder eine Substanz, der bzw. die in Abwesenheit der Zusammensetzung zur Verminderung von Gerüchen bei der Verwendung einen Geruch abgibt.

6. Zusammensetzung nach Anspruch 5, wobei der Gegenstand oder die Substanz mindestens ein Gegenstand bzw. mindestens eine Substanz, ausgewählt aus der Gruppe bestehend aus Körperpflegegegenständen, Fußpulvern, Waschmitteln, Haustierstreu, Reinigungsprodukten und Deodorantien, ist.

7. Zusammensetzung nach Anspruch 1, enthaltend etwa (i) 33 bis etwa 99% Säure, (ii) etwa 0,5 bis etwa 40% Metall oder Metalloxid und (iii) etwa 5 bis etwa 50% synthetischer Zeolith, wobei die Summe von (i), (ii) und (iii) 100% beträgt.

8. Zusammensetzung nach Anspruch 7, enthaltend etwa 88 bis etwa 89,5% Säure, etwa 0,5 bis etwa 2% Metall oder Metalloxid und etwa 9 bis etwa 11% synthetischer Zeolith.

9. Zusammensetzung nach Anspruch 7, enthaltend etwa 90 bis etwa 94% Säure, etwa 1 bis etwa 3% Metall oder Metalloxid und etwa 5 bis etwa 7% synthetischer Zeolith.

10. Zusammensetzung nach Anspruch 1, wobei die Säure und das Metalloxid oder Metall zusammen im Bereich von etwa 24% bis etwa 99,7% der gesamten Zusammensetzung liegen und der Zeolith im Bereich von etwa 0,3 bis etwa 76% der gesamten Zusammensetzung liegt.

11. Zusammensetzung nach Anspruch 10, wobei die Säure und das Metalloxid oder das Metall zusammen im Bereich von etwa 50% bis etwa 98% der gesamten Zusammensetzung liegen und der Zeolith im Bereich von etwa 2% bis etwa 50% der gesamten Zusammensetzung liegt.

12. Zusammensetzung nach Anspruch 1, des Weiteren umfassend ein Verdünnungsmittel.

13. Zusammensetzung nach Anspruch 12, wobei das Verdünnungsmittel Natriumbicarbonat oder ein natürlicher Zeolith ist.

14. Zusammensetzung nach Anspruch 13, wobei das Verdünnungsmittel Clinoptilolit ist.

15. Zusammensetzung nach Anspruch 14, enthaltend etwa 30 bis 38% Asparaginsäure oder Fumarsäure, etwa 0,5 bis 1% ZnO, etwa 1 bis 2% Zeolith und etwa 60 bis 67% Clinoptilolit.

16. Geruchsregulierter Gegenstand, umfassend:
eine wirksame Menge einer geruchsvermindernden Zusammensetzung nach einem der Ansprüche 1 bis 4 oder 7 bis 15; und
einen Gegenstand, der in Abwesenheit der geruchsvermindernden Zusammensetzung bei der Anwendung einen Geruch abgibt in Kontakt mit der geruchsvermindernden Zusammensetzung.

17. Gegenstand nach Anspruch 16, wobei der geruchsabgebende Gegenstand ausgewählt ist aus der Gruppe bestehend aus Einlagen, Papiertuch, Abwasserklärteichen ("lagoons"), Bandagen, Verbänden, chirurgischen Schwämmen, Körperpflegegegenständen, Reinigungsprodukten, Raumdesodorantien, Fahrzeugdesodorantien und Mülltüten.

18. Verfahren zur Verminderung von Gerüchen, welches umfasst das Inkontaktbringen einer wirksamen Menge einer geruchsvermindernden Zusammensetzung nach einem der Ansprüche 1 bis 4 oder 7 bis 15, mit einem Gegenstand, der in Abwesenheit der geruchsvermindernden Zusammensetzung bei der Verwendung einen Geruch abgibt für eine Zeitdauer, die ausreichend ist um den Geruch wirksam zu entfernen; und Entfernen des abgegebenen Geruchs von dem geruchsabgebenden Gegenstand.

19. Verfahren nach Anspruch 18, wobei der geruchsabgebende Gegenstand aus der Gruppe bestehend aus Einlagen, Abwasserklärteichen, Tanks, Tierabfällen, Bandagen, Verbänden, chirurgischen Schwämmen, Tampons, Rindfleisch-, Geflügel- und Fisch-Tabletts, Körperpflegegegenständen, Fußpulvern, Waschmitteln, Haustierstreu, Reinigungsprodukten, Desodorantien, Bettzeug, Fußböden, Mülltonnen, Windeleimern, Kühlschränken, Fahrzeugen und Teppichen ausgewählt ist.

20. Verfahren zum Entfernen von Gerüchen aus einer geruchsabgebenden Umgebung, umfassend: das Inkontaktbringen einer wirksamen Menge einer geruchsvermindernden Zusammensetzung nach Anspruch 1 mit der geruchsabgebenden Umgebung; und Verstreichenlassen einer ausreichenden Zeit, damit die Zusammensetzung den Geruch entfernen kann.

21. Verfahren nach Anspruch 20, wobei die Stufe des Inkontaktbringens einer wirksamen Menge einer geruchsvermindernden Zusammensetzung mit der geruchsabgebenden Umgebung das Inkontaktbringen einer geruchsvermindernden Zusammensetzung mit der geruchsabgebenden Umgebung umfasst, wobei die Zusammensetzung in einem Gegenstand enthalten ist, der das Einschließen der Zusammensetzung gestattet.

22. Zusammensetzung zur Verminderung von Gerüchen, umfassend:
(a) 0,1 Gew.-% bis 99,7 Gew.-% von mindestens einer Säure, ausgewählt aus Adipinsäure, Asparaginsäure, Cyclohexan-1,1-dicarbonsäure, Cystin, Dimethylmalonsäure, Fumarsäure, Sorbinsäure, Glutarsäure, Methylbernsteinsäure, Itaconsäure oder Weinsäure;
(b) 0,1 Gew.-% bis 99,7 Gew.-% von mindestens einem synthetischen Zeolith, bei dem mindestens 90% der tetraedrischen Oxideinheiten SiO₂-Tetraeder sind, mit einer Kapazität für adsorbiertes Wasser von nicht mehr als 10 Gew.-%, gemessen bei 25°C und einem Wasserdampfdruck von 4,6 Torr, und mit einer Porenöffnung von mindestens 5,5 Å Durchmesser, wobei das ursprüngliche Hydratwasser im Wesentlichen entfernt worden ist; und
(c) 0,1 Gew.-% bis 99,7 Gew.-% einer Substanz, ausgewählt aus der Gruppe bestehend aus einem Metall, Metalloxid, einem Salz eines Metalls oder Metalloxids und jeder Kombination davon;
wobei die Summe von (a), (b) und (c) 100 Gew.-% beträgt.

23. Zusammensetzung nach Anspruch 22, wobei das Metalloxid ausgewählt ist aus der Gruppe bestehend aus Zinkoxid, Kupferoxid, Eisenoxid, Manganoxid, Zinnoxid und Silberoxid.

24. Zusammensetzung nach Anspruch 22, wobei das Metall ausgewählt ist aus der Gruppe bestehend aus Zink, Kupfer, Eisen, Mangan, Zinn und Silber.

25. Zusammensetzung nach Anspruch 22, wobei das Salz ausgewählt ist aus der Gruppe bestehend aus einem Salz von Zink, Kupfer, Eisen, Mangan, Zinn und Silber.

26. Zusammensetzung nach Anspruch 22, enthaltend etwa (i) 33 bis etwa 99% Säure, (ii) etwa 0,5 bis etwa 40% Metall, Metalloxid oder Salz von einem Metall oder Metalloxid und (iii) etwa 5 bis etwa 50 Gew.-% synthetischer Zeolith, wobei die Summe von (i), (ii) und (iii) 100% beträgt.

27. Zusammensetzung nach Anspruch 26, enthaltend etwa 88 bis etwa 89,5% Säure, etwa 0,5 bis etwa 2% Metall, Metalloxid oder Salz von einem Metall oder Metalloxid und etwa 9 bis etwa 11% synthetischer Zeolith.

28. Zusammensetzung nach Anspruch 26, enthaltend etwa 90 bis etwa 94% Säure, etwa 1 bis etwa 3% Metall, Metalloxid oder Salz von einem Metall oder Metalloxid und etwa 5 bis etwa 7% synthetischer Zeolith.

29. Zusammensetzung nach Anspruch 22, wobei die Säure und das Metalloxid, Metall oder Salz von einem Metall oder Metalloxid zusammen im Bereich von etwa 24 bis 99,7% der gesamten Zusammensetzung liegen und der Zeolith im Bereich von etwa 0,3 bis etwa 76% der gesamten Zusammensetzung liegt.

30. Zusammensetzung nach Anspruch 29, wobei die Säure und das Metalloxid, Metall oder Salz von Metall oder Metalloxid zusammen im Bereich von etwa 50% bis etwa 98% der gesamten Zusammensetzung liegen und der Zeolith im Bereich von etwa 2% bis etwa 50% der gesamten Zusammensetzung liegt.

31. Zusammensetzung nach Anspruch 22, des Weiteren umfassend ein Verdünnungsmittel.

32. Zusammensetzung nach Anspruch 31, wobei das Verdünnungsmittel Natriumbicarbonat oder ein natürlicher Zeolith ist.

33. Zusammensetzung nach Anspruch 32, wobei das Verdünnungsmittel Clinoptilolit ist.

34. Zusammensetzung nach Anspruch 33, enthaltend etwa 30 bis 38% Asparaginsäure oder Fumarsäure, etwa 0,5 bis 1% ZnO, etwa 1 bis 2% Zeolith und etwa 60 bis 67% Clinoptilolit.

35. Zusammensetzung nach Anspruch 22, wobei die mindestens eine Säure ausgewählt ist aus Asparaginsäure, Fumarsäure, Sorbinsäure, Weinsäure oder Adipinsäure.

36. Zusammensetzung nach Anspruch 35, wobei das Metalloxid oder Salz von einem Metall ausgewählt ist aus der Gruppe bestehend aus Zinkoxid und Zinksulfat.

37. Geruchsregulierter Gegenstand, umfassend:
eine wirksame Menge einer geruchsvermindernden Zusammensetzung nach einem der Ansprüche 22 bis 34 oder 36 und
einen Gegenstand, der in Abwesenheit der geruchsvermindernden Zusammensetzung bei der Verwendung einen Geruch abgibt in Kontakt mit der geruchsvermindernden Zusammensetzung.

38. Gegenstand nach Anspruch 37, wobei der geruchsabgebende Gegenstand ausgewählt ist aus der Gruppe bestehend aus Einlagen, Papiertuch, Abwasserklärteichen, Bandagen, Verbänden, chirurgischen Schwämmen, Körperpflegegegenständen, Reinigungsprodukten, Raumdesodorantien, Fahrzeugdesodorantien und Mülltüten.

39. Verfahren zur Verminderung von Gerüchen, umfassend das Inkontaktbringen einer wirksamen Menge einer geruchsvermindernden Zusammensetzung nach einem der Ansprüche 22 bis 34
mit einem Gegenstand, der in Abwesenheit der geruchsvermindernden Zusammensetzung bei der Verwendung einen Geruch abgibt für eine Zeitdauer, die ausreichend ist um den Geruch wirksam zu entfernen; und Entfernen des abgegebenen Geruchs von dem geruchsabgebenden Gegenstand.

40. Verfahren nach Anspruch 39, wobei der geruchsabgebende Gegenstand aus der Gruppe bestehend aus Einlagen, Abwasserklärteichen, Tanks, Tierabfällen, Bandagen, Verbänden, chirurgischen Schwämmen, Tampons, Rindfleisch-, Geflügel- und Fisch-Tabletts, Körperpflegegegenständen, Fußpulvern, Waschmitteln, Haustierstreu, Reinigungsprodukten, Desodorantien, Bettzeug, Fußböden, Mülltonnen, Windeleimern, Kühlschränken, Fahrzeugen und Teppichen ausgewählt ist.

41. Verfahren zum Entfernen von Gerüchen aus einer geruchsabgebenden Umgebung, umfassend: das Inkontaktbringen einer wirksamen Menge einer geruchsvermindernden Zusammensetzung nach Anspruch 22
mit der geruchsabgebenden Umgebung; und Verstreichenlassen einer ausreichenden Zeit, damit die Zusammensetzung den Geruch entfernen kann.

42. Verfahren nach Anspruch 41, wobei die Stufe des Inkontaktbringens einer wirksamen Menge einer geruchsvermindernden Zusammensetzung mit der geruchsabgebenden Umgebung das Inkontaktbringen einer geruchsvermindernden Zusammensetzung mit der geruchsabgebenden Umgebung umfasst, wobei die Zusammensetzung in einem Gegenstand enthalten ist, der das Einschließen der Zusammensetzung gestattet.

## Revendications

1. Composition destinée à réduire les odeurs comprenant :
(a) de 0,1 % en poids à 99,7 % en poids d'au moins un acide ayant un pKa supérieur à 2,9 et inférieur à 6, une solubilité dans l'eau inférieure à 45 g/100 g de H₂O, et une D.L.50 orale chez le rat supérieure à 2200 mg/kg ;
(b) de 0,1 % en poids à 99,7 % en poids d'au moins une zéolithe synthétique ayant au moins 90 pour cent de ses unités d'oxyde tétraédriques sous forme de SiO₂ tétraédrique, une capacité d'absorption d'eau inférieure ou égale à 10 pour cent en poids lorsque mesurée à 25 °C et à une pression de vapeur d'eau de 4,6 torrs, et des ouvertures de pores d'au moins 5,5 Å de diamètre, où l'eau d'hydratation d'origine a été substantiellement éliminée ; et
(c) de 0,1 % en poids à 99,7 % en poids d'une substance sélectionnée dans le groupe constitué d'un métal, un oxyde de métal et de toute combinaison de ceux-ci ;
dans laquelle la somme de (a), (b) et (c) correspond à 100 % en poids.

2. Composition selon la revendication 1, dans laquelle ledit acide est au moins un acide sélectionné dans le groupe constitué d'un acide organique, un acide aminé et toute combinaison de ceux-ci.

3. Composition selon la revendication 1, dans laquelle ledit oxyde de métal est sélectionné dans le groupe constitué de l'oxyde de zinc, l'oxyde de cuivre, l'oxyde de fer, l'oxyde de manganèse, l'oxyde d'étain et l'oxyde d'argent.

4. Composition selon la revendication 1, dans laquelle ledit métal est sélectionné dans le groupe constitué du zinc, du cuivre, du fer, du manganèse, de l'étain et de l'argent.

5. Composition selon la revendication 1 comprenant en outre un article ou une substance qui, en l'absence de ladite composition destinée à réduire les odeurs, émet une odeur au cours de l'utilisation.

6. Composition selon la revendication 5, dans laquelle ladite substance ou ledit article est au moins un sélectionné dans le groupe constitué d'articles de soins personnels, de poudres pour les pieds, de préparations de blanchissage, de litières, de produits d'entretien et de désodorisants.

7. Composition selon la revendication 1 ayant d'environ (i) 33 à environ 99 % d'acide, (ii) d'environ 0,5 à environ 40 % de métal ou d'oxyde de métal, et (iii) d'environ 5 à environ 50 % de zéolithe synthétique, dans laquelle la somme de (i), (ii) et (iii) est 100 %.

8. Composition selon la revendication 7 ayant d'environ 88 à environ 89,5 % d'acide, d'environ 0,5 à environ 2 % de métal ou d'oxyde de métal et d'environ 9 à environ 11 % de zéolithe synthétique.

9. Composition selon la revendication 7 ayant d'environ 90 à environ 94 % d'acide, d'environ 1 à environ 3 % de métal ou d'oxyde de métal et d'environ 5 à environ 7 % de zéolithe synthétique.

10. Composition selon la revendication 1, dans laquelle ledit acide et ledit oxyde de métal ou métal combinés représentent d'environ 24 % à environ 99,7 % de la composition totale et ladite zéolithe représente d'environ 0,3 % à environ 76 % de la composition totale.

11. Composition selon la revendication 10, dans laquelle ledit acide et ledit oxyde de métal ou métal combinés représentent d'environ 50 % à environ 98 % de la composition totale et ladite zéolithe représente d'environ 2 % à environ 50 % de la composition totale.

12. Composition selon la revendication 1 comprenant en outre un diluant.

13. Composition selon la revendication 12, dans laquelle ledit diluant est le bicarbonate de sodium ou une zéolithe naturelle.

14. Composition selon la revendication 13, dans laquelle ledit diluant est une clinoptilolite.

15. Composition selon la revendication 14 ayant d'environ 30 à 38 % d'acide aspartique ou d'acide fumarique, d'environ 0,5 à 1 % de ZnO, d'environ 1 à 2 % de zéolithe, et d'environ 60 à 67 % de clinoptilolite.

16. Article protégé contre les odeurs comprenant :
une quantité efficace d'une composition réduisant les odeurs selon l'une quelconque des revendications 1 à 4 ou 7 à 15 ; et
un article qui, en l'absence de ladite composition réduisant les odeurs, émet une odeur au cours de l'utilisation, en contact avec ladite composition réduisant les odeurs.

17. Article selon la revendication 16, dans lequel ledit article émettant une odeur est sélectionné dans le groupe constitué de serviettes hygiéniques, de mouchoirs en papier, de lagons, de bandages, de pansements, de tampons pour dissection, d'articles de soins personnels, de produits d'entretien, de désodorisants d'ambiance, de désodorisants de voiture, et de sacs à ordures.

18. Procédé destiné à réduire les odeurs comprenant la mise en contact d'une quantité efficace d'une composition réduisant les odeurs selon l'une quelconque des revendications 1 à 4 ou 7 à 15, avec un article qui, en l'absence de ladite composition réduisant les odeurs, émet une odeur au cours de l'utilisation, pendant un temps suffisant pour éliminer efficacement ladite odeur, et l'élimination de ladite odeur émise par ledit article émettant une odeur.

19. Procédé selon la revendication 18, dans lequel ledit article émettant une odeur est sélectionné dans le groupe constitué de serviettes hygiéniques, de lagons, de cuves, de déchets animaux, de bandages, de pansements, de tampons pour dissections, de dispositifs cataméniaux, de plateaux de boeuf, de volaille et de poisson, d'articles de soins personnels, de poudres pour les pieds, de préparations de blanchissage, de litières, de produits d'entretien, de désodorisants, de literies, de sols, de poubelles, de sacs de couches, de réfrigérateurs, de véhicules, et de tapis.

20. Procédé d'élimination d'odeurs d'un environnement émettant des odeurs comprenant : la mise en contact d'une quantité efficace d'une composition réduisant les odeurs selon la revendication 1 avec ledit environnement émettant des odeurs ; et le fait de laisser s'écouler un temps suffisant pour que ladite composition élimine l'odeur.

21. Procédé selon la revendication 20, dans lequel ladite étape de mise en contact d'une quantité efficace d'une composition réduisant les odeurs avec ledit environnement émettant des odeurs comprend la mise en contact d'une composition réduisant les odeurs, dans laquelle ladite composition est contenue dans un article qui permet de contenir ladite composition, avec ledit environnement émettant des odeurs.

22. Composition réduisant des odeurs comprenant :
(a) de 0,1 % en poids à 99,7 % en poids d'au moins un acide sélectionné parmi l'acide adipique, aspartique, cyclohexane-1:1-dicarboxylique, la cystine, l'acide diméthylmalonique, fumarique, sorbique, glutarique, méthylsuccinique, itaconique, ou tartrique ;
(b) de 0,1 % en poids à 99,7 % en poids d'au moins une zéolithe synthétique ayant au moins 90 pour cent de ses unités d'oxyde tétraédriques sous forme de SiO₂ tétraédrique, une capacité d'absorption d'eau inférieure ou égale à 10 pour cent en poids lorsque mesurée à 25 °C et à une pression de vapeur d'eau de 4,6 torrs, et des ouvertures de pores d'au moins 5,5 Å de diamètre, où l'eau d'hydratation d'origine a été substantiellement éliminée ; et
(c) de 0,1 % en poids à 99,7 % en poids d'une substance sélectionnée dans le groupe constitué d'un métal, un oxyde de métal, un sel d'un métal ou d'un oxyde de métal, et de toute combinaison de ceux-ci ;
dans laquelle la somme de (a), (b) et (c) correspond à 100 % en poids.

23. Composition selon la revendication 22, dans laquelle ledit oxyde de métal est sélectionné dans le groupe constitué de l'oxyde de zinc, l'oxyde de cuivre, l'oxyde de fer, l'oxyde de manganèse, l'oxyde d'étain et l'oxyde d'argent.

24. Composition selon la revendication 22, dans laquelle ledit métal est sélectionné dans le groupe constitué du zinc, du cuivre, du fer, du manganèse, de l'étain et de l'argent.

25. Composition selon la revendication 22, dans laquelle ledit sel est sélectionné dans le groupe constitué d'un sel de zinc, de cuivre, de fer, de manganèse, d'étain et d'argent.

26. Composition selon la revendication 22 ayant d'environ (i) 33 à environ 99 % d'acide, (ii) d'environ 0,5 à environ 40 % de métal, d'oxyde de métal ou de sel d'un métal ou d'un oxyde de métal, et (iii) d'environ 5 à environ 50 % de zéolithe synthétique, dans laquelle la somme de (i), (ii) et (iii) est 100 %.

27. Composition selon la revendication 26 ayant d'environ 88 à environ 89,5 % d'acide, d'environ 0,5 à environ 2 % de métal, d'oxyde de métal ou de sel d'un métal ou d'un oxyde de métal, et d'environ 9 à environ 11 % de zéolithe synthétique.

28. Composition selon la revendication 26 ayant d'environ 90 à environ 94 % d'acide, d'environ 1 à environ 3 % de métal, d'oxyde de métal ou de sel d'un métal ou d'un oxyde de métal, et d'environ 5 à environ 7 % de zéolithe synthétique.

29. Composition selon la revendication 22, dans laquelle ledit acide et ledit oxyde de métal, métal, ou sel de métal ou d'oxyde de métal combinés représentent d'environ 24 % à environ 99,7 % de la composition totale et ladite zéolithe représente d'environ 0,3 % à environ 76 % de la composition totale.

30. Composition selon la revendication 29, dans laquelle ledit acide et ledit oxyde de métal, métal ou sel de métal ou d'oxyde de métal combinés représentent d'environ 50 % à environ 98 % de la composition totale et ladite zéolithe représente d'environ 2 % à environ 50 % de la composition totale.

31. Composition selon la revendication 22 comprenant en outre un diluant.

32. Composition selon la revendication 31, dans laquelle ledit diluant est le bicarbonate de sodium ou une zéolithe naturelle.

33. Composition selon la revendication 32, dans laquelle ledit diluant est une clinoptilolite.

34. Composition selon la revendication 33 ayant d'environ 30 à 38 % d'acide aspartique ou d'acide fumarique, d'environ 0,5 à 1 % de ZnO, d'environ 1 à 2 % de zéolithe, et d'environ 60 à 67 % de clinoptilolite.

35. Composition selon la revendication 22, dans laquelle ledit au moins un acide est sélectionné parmi l'acide aspartique, fumarique, sorbique, tartrique ou adipique.

36. Composition selon la revendication 35, dans laquelle ledit oxyde de métal ou sel d'un métal est sélectionné dans le groupe constitué de l'oxyde de zinc et du sulfate de zinc.

37. Article protégé contre les odeurs comprenant :
une quantité efficace d'une composition réduisant les odeurs selon l'une quelconque des revendications 22 à 34 ou 36 ; et
un article qui, en l'absence de ladite composition réduisant les odeurs, émet une odeur au cours de l'utilisation, en contact avec ladite composition réduisant les odeurs.

38. Article selon la revendication 37, dans lequel ledit article émettant une odeur est sélectionné dans le groupe constitué de serviettes hygiéniques, de mouchoirs en papier, de lagons, de bandages, de pansements, de tampons pour dissection, d'articles de soins personnels, de produits d'entretien, de désodorisants d'ambiance, de désodorisants de voiture, et de sacs à ordures.

39. Procédé destiné à réduire les odeurs comprenant la mise en contact d'une quantité efficace d'une composition réduisant les odeurs selon l'une quelconque des revendications 22 à 34 avec un article qui, en l'absence de ladite composition réduisant les odeurs, émet une odeur au cours de l'utilisation, pendant un temps suffisant pour éliminer efficacement ladite odeur, et l'élimination de ladite odeur émise par ledit article émettant une odeur.

40. Procédé selon la revendication 39, dans lequel ledit article émettant une odeur est sélectionné dans le groupe constitué de serviettes hygiéniques, de lagons, de cuves, de déchets animaux, de bandages, de pansements, de tampons pour dissections, de dispositifs cataméniaux, de plateaux de boeuf, de volaille et de poisson, d'articles de soins personnels, de poudres pour les pieds, de préparations de blanchissage, de litières, de produits d'entretien, de désodorisants, de literies, de sols, de poubelles, de sacs de couches, de réfrigérateurs, de véhicules, et de tapis.

41. Procédé d'élimination d'odeurs d'un environnement émettant des odeurs comprenant : la mise en contact d'une quantité efficace d'une composition réduisant les odeurs selon la revendication 22 avec ledit environnement émettant des odeurs ; et le fait de laisser s'écouler un temps suffisant pour que ladite composition élimine l'odeur.

42. Procédé selon la revendication 41, dans lequel ladite étape de mise en contact d'une quantité efficace d'une composition réduisant les odeurs avec ledit environnement émettant des odeurs comprend la mise en contact d'une composition réduisant les odeurs, dans laquelle ladite composition est contenue dans un article qui permet de contenir ladite composition, avec ledit environnement émettant des odeurs.
